# EUROPEAN PATENT APPLICATION

(11) **EP 4 057 294 A1**
(43) Date of publication of application: **14.09.2022**
(21) Application number: 21208982.5
(22) Date of filing: 18.11.2021
(51) Int. Cl.: G16H 40/20, G06Q 50/22, G16H 50/30, G16H 50/80

(54) **SYSTEM TO MANAGE MOVEMENT WITHIN A BUILDING TO REDUCE PERSONAL RISK**

(30) Priority: 09.03.2021 US 202117195927
(71) Applicant: Otis Elevator Company, Farmington, Connecticut 06032 (US)
(72) Inventor: MIRIYALA, Pradeep, 500081 Telegana (IN); DHAR, Abhinav, 500081 Telangana (IN)
(74) Representative: Dehns

(57) **Abstract**

An illustrative example embodiment of a method of managing risk associated with at least one individual (42) moving within a building (22) includes: determining a beginning location and a destination of an individual, wherein the beginning location and the destination are on different levels of the building; determining a risk contribution of the individual based on an indication of at least one characteristic of the individual; determining a plurality of routes between the beginning location and the destination; determining a risk factor of each of the plurality of routes based on the risk contribution of the individual and other risk information regarding the respective routes; and determining a recommended route from among the plurality of routes for the individual to arrive at the destination, wherein the risk factor of the recommended route is lower than the risk factor of at least one other one of the plurality of routes.

## Description

### BACKGROUND

There are circumstances in which it can be useful or important to direct people to move along certain pathways or routes within a building. For example, individual safety or health may depend on maintaining distance between individuals within the same building.

During an epidemic it can be challenging to move throughout some buildings without incurring some risk of exposure to the disease. Getting to certain levels within a building may require using an elevator or escalator, which can place occupants in proximity to one another. The risk of infection may be reduced by avoiding being in close proximity to others who may be infected. Suggesting or controlling the routes individuals take between different levels in a building can facilitate such protective measures.

### SUMMARY

An illustrative example embodiment of a method of managing risk associated with at least one individual moving within a building includes: determining a beginning location and a destination of an individual, wherein the beginning location and the destination are on different levels of the building; determining a risk contribution of the individual based on an indication of at least one characteristic of the individual; determining a plurality of routes between the beginning location and the destination; determining a risk factor of each of the plurality of routes based on the risk contribution of the individual and other risk information regarding the respective routes; and determining a recommended route from among the plurality of routes for the individual to arrive at the destination, wherein the risk factor of the recommended route is lower than the risk factor of at least one other one of the plurality of routes.

In addition to one or more of the features described above, or as an alternative, at least a first one of the plurality of routes includes an elevator and at least a second one of the plurality of routes includes at least one stairway or escalator, and determining the recommended route includes prioritizing the first one of the routes based on the risk contribution of the individual satisfying at least one criterion.

In addition to one or more of the features described above, or as an alternative, the method includes prioritizing the first one of the routes over the second one of the routes when the at least one criterion corresponds to the individual contributing to an increased risk of spreading infection within the building.

In addition to one or more of the features described above, or as an alternative, the method includes when the first one of the routes is the recommended route, automatically scheduling a call for the elevator to take the individual along a corresponding portion of the recommended route to the destination.

In addition to one or more of the features described above, or as an alternative, determining the risk contribution of the individual comprises receiving information regarding the at least one characteristic of the individual in association with receiving information regarding the beginning location and the destination, and the at least one characteristic of the individual corresponds to a likelihood that the individual is infected with a contagious disease.

In addition to one or more of the features described above, or as an alternative, the information regarding the at least one characteristic of the individual is based on an output of a contract tracing application.

In addition to one or more of the features described above, or as an alternative, the determined risk contribution of the individual indicates that it is unlikely that the individual has contracted a contagious disease, the risk factor corresponds to a risk that the individual will be exposed to the contagious disease or will encounter at least one other individual that is likely infected with the contagious disease, and the recommended route reduces or minimizes the risk that the individual will be exposed to the contagious disease while moving toward the destination.

In addition to one or more of the features described above, or as an alternative, determining a relationship between the risk factor of the recommended route and at least one threshold; when the risk factor of the recommended route is below a first threshold, notify the individual that travel to the destination is approved with low risk and automatically schedule an elevator call if an elevator is included on the recommended route; when the risk factor of the recommended route is above the first threshold and below a second threshold, notify the individual that travel to the destination at a current time has an associated risk of infection, determine at least one alternative route at a different time, notify the individual of the at least one alternative route, and receive a selection from the individual of the recommended route or the alternative route; and when the risk factor of the recommended route is above the second threshold, notify the individual that it is potentially unsafe for the individual to travel to the destination at the current time.

In addition to one or more of the features described above, or as an alternative, the determined risk contribution of the individual indicates that it is likely or certain that the individual has contracted a contagious disease, the risk factor corresponds to a risk that the individual will expose others along the respective route to the contagious disease, and the recommended route reduces or minimizes the risk that the individual will expose others to the contagious disease while moving toward the destination.

In addition to one or more of the features described above, or as an alternative, determining the recommended route comprises avoiding any of the plurality of routes that includes the individual using an escalator and the method comprises automatically scheduling an elevator call for any elevator included on the recommended route; assigning an unsafe route status to the recommended route beginning at a time when the individual is expected to begin taking the recommended route; scheduling a cleaning or sanitizing of at least portions of the recommended route; and changing the unsafe route status to an available route status based on completion of the scheduled cleaning or sanitizing of at least the portions of the recommended route.

An illustrative example embodiment of a system for managing risk associated with at least one individual moving within a building includes a receiver configured to receive an indication of at least one characteristic of an individual and an indication of a beginning location and a destination of the individual. The beginning location and the destination are on different levels of the building. A processor is configured to determine a risk contribution of the individual based on the indication of the at least one characteristic of the individual, determine a plurality of routes between the beginning location and the destination, determine a risk factor of each of the plurality of routes based on the risk contribution of the individual and other risk information regarding the respective routes, and determine a recommended route from among the plurality of routes for the individual to arrive at the destination, wherein the risk factor of the recommended route is lower than the risk factor of at least one other one of the plurality of routes.

In addition to one or more of the features described above, or as an alternative, at least a first one of the plurality of routes includes an elevator and at least a second one of the plurality of routes includes at least one stairway or escalator, and the processor is configured to determine the recommended route by prioritizing the first one of the routes based on the risk contribution of the individual satisfying at least one criterion.

In addition to one or more of the features described above, or as an alternative, the at least one criterion corresponds to the individual contributing to an increased risk of spreading infection within the building.

In addition to one or more of the features described above, or as an alternative, when the first one of the routes is the recommended route, the processor automatically schedules a call for the elevator to take the individual along a corresponding portion of the recommended route to the destination.

In addition to one or more of the features described above, or as an alternative, the at least one characteristic of the individual corresponds to a likelihood that the individual is infected with a contagious disease.

In addition to one or more of the features described above, or as an alternative, the information regarding the at least one characteristic of the individual is based on an output of a contract tracing application.

In addition to one or more of the features described above, or as an alternative, the determined risk contribution of the individual indicates that it is unlikely that the individual has contracted a contagious disease, the risk factor corresponds to a risk that the individual will be exposed to the contagious disease or will encounter at least one other individual that is likely infected with the contagious disease, and the recommended route reduces or minimizes the risk that the individual will be exposed to the contagious disease while moving toward the destination.

In addition to one or more of the features described above, or as an alternative, the processor is configured to determine a relationship between the risk factor of the recommended route and at least one threshold; when the risk factor of the recommended route is below a first threshold, notify the individual that travel to the destination is approved with low risk and automatically schedule an elevator call if an elevator is included on the recommended route; when the risk factor of the recommended route is above the first threshold and below a second threshold, notify the individual that travel to the destination at a current time has an associated risk of infection, determine at least one alternative route at a different time, notify the individual of the at least one alternative route, and receive a selection from the individual of the recommended route or the alternative route; and when the risk factor of the recommended route is above the second threshold, notify the individual that it is potentially unsafe for the individual to travel to the destination at the current time.

In addition to one or more of the features described above, or as an alternative, the determined risk contribution of the individual indicates that it is likely or certain that the individual has contracted a contagious disease, the risk factor corresponds to a risk that the individual will expose others along the respective route to the contagious disease, and the recommended route reduces or minimizes the risk that the individual will expose others to the contagious disease while moving toward the destination.

In addition to one or more of the features described above, or as an alternative, determining the recommended route comprises avoiding any of the plurality of routes that includes the individual using an escalator and the processor is configured to automatically schedule an elevator call for any elevator included on the recommended route; assign an unsafe route status to the recommended route beginning at a time when the individual is expected to begin taking the recommended route; schedule a cleaning or sanitizing of at least portions of the recommended route; and change the unsafe route status to an available route status based on completion of the scheduled cleaning or sanitizing of at least the portions of the recommended route.

The various features and advantages of at least one disclosed example embodiment will become apparent to those skilled in the art from the following detailed description. The drawings that accompany the detailed description can be briefly described as follows.

### BRIEF DESCRIPTION OF THE DRAWINGS

Figure 1 schematically illustrates selected portions of an example embodiment of a system for managing movement of people within a building in a manner that reduces risk.
Figure 2 is a flow chart diagram summarizing an example embodiment of a process of recommending a route for an individual to take within a building.
Figure 3 is a flow chart diagram summarizing an example embodiment of a process of recommending a route within a building for an individual who does not contribute to a risk.
Figure 4 is a flow chart diagram summarizing an example embodiment of a process of recommending a route within a building for an individual who introduces additional risk.

### DETAILED DESCRIPTION

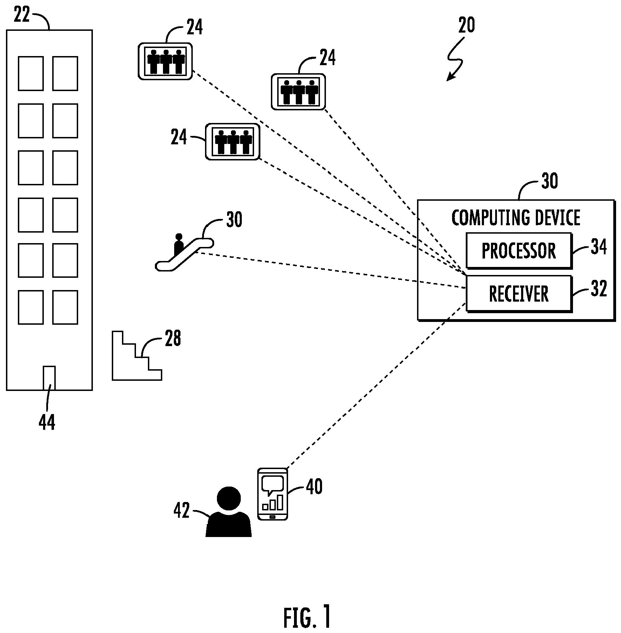

Figure 1 schematically illustrates selected portions of a system 20 for managing movement of people within a building 22 that includes multiple levels. There are several ways an individual may travel or move between different levels in the building 22 including elevators 24, at least one escalator 26 and at least one stairway 28.

A computing device 30 includes a receiver 32 and a processor 34. The computing device is schematically illustrated as a single device for discussion purposes but may be realized using various combinations of computing equipment including, for example, cloud computing resources. The computing device 30 may be located at the site of the building 22 or at a remote location.

The receiver 32 is configured to receive information regarding the status of the elevators 24 and the escalator 26, which may be obtained, for example, from an existing or previously installed remote monitoring system. The receiver is also configured to communicate with personal devices, such as a mobile station or smart phone 40 used by an individual 42 who desires to move between a beginning location in the building 22 and a destination in the building 22. The direction of movement of the individual 42 in the building 22 may vary depending on the circumstances. For example, the intended travel may be upward from a main entrance 44 to an upper level of the building 22, downward from an upper level to the lobby, or in either direction between levels within the building 22.

The processor 34 obtains information through the receiver regarding conditions within the building 22, such as the current status of the elevators 24 and escalator 26. In some embodiments, the building 22 includes or is associated with a building management system that can provide information to the processor 34 regarding conditions in the building 22. For example, if such information is available, a building management system may monitor conditions such as occupancy or floor closures. There are a variety of ways in which the processor 34 can obtain information regarding conditions in the building 22 that are pertinent to the processor 34 making decisions regarding recommending a route to the individual 42.

Managing movement of people within the building 22 to avoid spreading an infectious disease is discussed as part of the example embodiments and considered characteristics of the individual 42 correspond to a likelihood that the individual 42 is infected with the contagious disease. A variety of characteristics may be important or useful in different circumstances where other types of risk are under consideration. Those skilled in the art who have this description will be able to apply the teachings of this document to their particular situation and select appropriate characteristics to address the risk under consideration in their particular implementation.

The processor 34 obtains information regarding at least one characteristic of the individual 42, which may be based on data from at least one source. For example, information may be sent by the mobile station 40 to the receiver 32 or otherwise provided by the individual 42. In the illustrated embodiment, the individual 42 uses an application on the mobile station 40 to communicate an indication of the characteristic(s) useful to the processor 34 for recommending a route for the individual to take within the building 22. One example application on the mobile station 40 provides contact tracing information over a recent period. Known contact tracing applications, such as those developed by government agencies or the application programming interface (API) deployed by Google and Apple, allow for determining a risk that the individual 42 has been exposed to or infected with a contagious disease. Such information may be used by the processor 34. In some embodiments, the individual 42 knows whether she is currently infected, contagious, or healthy, and provides information regarding that status through the mobile station 40 to the receiver 32. In other embodiments, the individual uses another device, such as a thermometer, to obtain or provide pertinent characteristic information, such as a current body temperature.

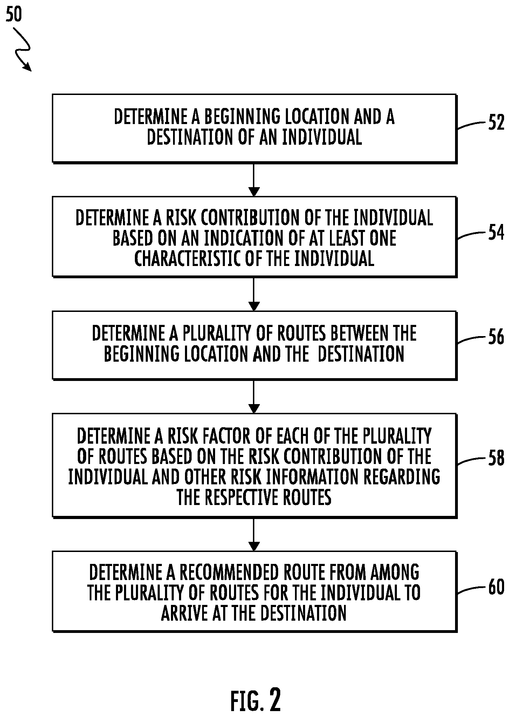

Figure 2 is a flow chart diagram 50 that summarizes an example method of managing movement within the building 22 of the individual 42 after receiving an indication of the individual's intentions. At 52, the processor 34 uses received information about the individual 42, such as that obtained by communicating with the mobile station 40, to determine the beginning location and the destination of the individual 42. The beginning location and the destination are on different levels of the building 22, and the individual will have to move or travel using the stairs 28, an elevator 24, the escalator 26 or a combination of them.

At 54, the processor 34 determines a risk contribution of the individual 42 based on an indication of the characteristic(s) provided by the individual 42. The characteristic may be information from a contact tracing application on the mobile station 40 or other health-related information from the individual. For example, the contract tracing application may provide a risk assessment or rating and the individual 42 sends the risk assessment or rating information to the receiver 32. Additionally, the characteristic information may include negative test results, a positive diagnosis of infection, or whether the individual has received a vaccination.

At 56, the processor 34 determines a plurality of routes within the building 22 between the beginning location and the destination. For example, the processor 34 has associated memory or is otherwise able to access information regarding the building configuration or layout to determine the plurality of routes. In some embodiments, a set of routes between expected beginning locations and destinations are predetermined and stored in memory, such as a look-up table.

In some embodiments, the building 22 is represented by a network graph, such as a dynamic directed-weighted graph including the elevators 24, the escalator 26 and the stairs 28 as nodes. Possible beginning locations and possible destinations can also be nodes in the building graph. The edges between nodes represent travel between the nodes. The edges are weighted based on a combination of the distance or travel time along the portion of the building corresponding the edge and a risk factor or safety level along the corresponding portion of the route. Techniques for developing and utilizing such a network graph or other building representations or models are known and outside the scope of this description. Those skilled in the art who have the benefit of this description will be able to select an appropriate building model for their particular implementation.

At 58, the processor 34 uses information regarding a status of the portions of the building along the respective routes to determine a risk factor for each route between the beginning location and the destination of the individual 42. The risk factor in some example embodiments is different for a healthy individual than for an infected or likely infected individual. For example, when the individual 42 is healthy, the risk factor of each route corresponds to a risk that the individual 42 will be exposed to the infectious disease or encounter others who are infected. When the individual 42 is infected, the risk factor of each route corresponds to a risk that the individual 42 spread the disease by potentially contaminating a portion of the building or encountering others that are not sick. In other words, the risk factor has a different meaning or significance depending on whether the individual 42 is healthy or sick. The processor 32 is programmed or otherwise configured to use the appropriate risk factor determination for each individual 42 based on the determined risk contribution of that individual.

The status information used to determine the risk factor in example embodiments is based on information previously provided to the processor 34 regarding the risk contributions of people within the building 22 and moving along the respective routes. Some buildings include sensors that provide additional information to the processor 34 regarding the status of the respective routes, such as current occupancy or traffic information. The status may be updated on a schedule and in response to particular events. For example, the building model and information regarding the status of the routes may be updated every ten minutes. Another way in which the status information may be updated is based on detecting that an individual within the building 22 or along a route has a fever. Additionally, whenever an individual who is known to be infected or otherwise presents a high risk of spreading infection, the status can be updated based on such an individual entering the building 22 or following one of the known routes. In some embodiments, such routes will not be considered available for further travel until any affected area is appropriately cleaned or disinfected. That way, a route may be effectively closed to further traffic during cleaning. Another example event that may prompt a status update is when cleaning personnel clean or disinfect an area or route that was used by an individual known to be infected.

The manner in which the building and risk status information is modeled and maintained is outside the scope of this description. Those skilled in the art who have the benefit of this description will realize what model or technique will be useful to track or monitor the status of the building 22.

At 60, the processor 34 determines a recommended route from among the plurality of possible routes to the destination by selecting a route having a risk factor that is lower than the risk factor of another route. In some embodiments, the travel time along each route is considered as a factor for determining which route to recommend. In embodiments where the lowest risk factor is prioritized over travel time or another consideration, the processor 34 identifies which of the routes has the lowest risk factor and recommends that route.

As mentioned above, the risk factor is different depending on whether the individual 42 presents additional risk. When the information regarding the characteristic(s) of the individual 42 correspond to a healthy individual who has not had known exposure to the disease, that individual does not introduce additional risk of spreading infection. In that case, the risk factors determined at 58 represent or correspond to a risk to the individual 42 of encountering an infected individual or otherwise being exposed to the disease while moving between the beginning location and the individual's destination.

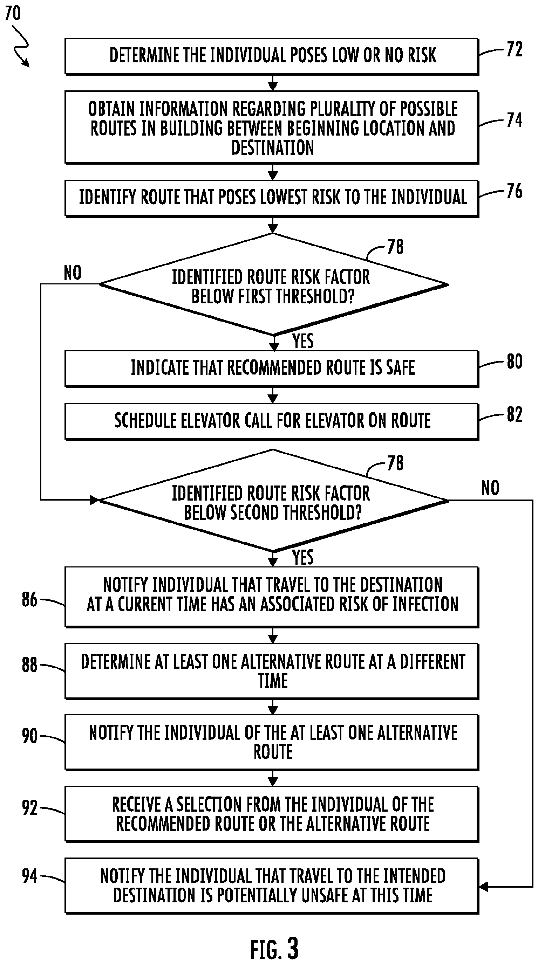

Figure 3 includes a flow chart diagram 70 that summarizes an example approach to recommend a route within the building 22 when the individual 42 does not pose a risk or contribute to an increased risk. At 72, the processor 34 uses the risk contribution determined at 54 (Figure 2) and determines that the individual 42 poses low or no risk of spreading infection. At 74, the processor 34 obtains information regarding a plurality of possible routes in the building 22 between the beginning location and the destination. The processor 34 identifies the route that poses the lowest risk to the individual 42 at 76. In some embodiments, the determination at 76 is based on identifying a fastest route with a risk factor that is lower than another route without necessarily requiring that the risk factor for such a route is the lowest of all risk factors for the possible routes.

The processor 34 compares the risk factor of the route identified at 76 to a first threshold at 78. If that risk factor is below the first threshold, the processor 34 indicates at 80 that the recommended route is safe. The indication may be provided to the individual 42 through the mobile station 40, for example. Alternatively, an appropriate interface in the building 22 may provide the indication.

If the recommended route includes an elevator 24, the example process includes automatically scheduling an elevator call at 82. Information regarding which elevator car to board may be provided to the individual 42 through the same interface used to provide the safe route indication at 80. The processor 34 updates the building model with information regarding the individual 42 traveling along the recommended route.

In the event that the risk factor of the recommended route is above the first threshold at 78, the processor 34 determines at 84 whether that risk factor is below a second threshold that is higher or greater than the first threshold. If the risk factor is below the second threshold, the processor continues at 86 to notify the individual 42 that travel to the destination at the current time includes some risk of infection. At 88, the processor 34 determines at least one alternative route at a different time. For example, it may be possible that one of the possible routes currently poses a higher risk than the recommended route but such a route will be clear of other individuals or cleaned within a few minutes. In such a case, the processor 34 obtains such information through the building model. If an alternative route is identified at 88, the processor notifies the individual 42 of the alternative route at 90.

In this example embodiment, the user or individual 42 has the option of selecting the recommended route or the alternative route. The processor 34 receives the individual's selection at 92 and updates the building model to reflect that individual 42 traveling along the selected route at an appropriate time.

In the illustrated example embodiment, if the risk factor of the route identified at 76 exceeds the second threshold at 84, the processor 34 determines that the movement of the individual 42 at the current time poses too high of a risk of infection for the processor 34 to recommend a route. At 94, the processor causes a notification to be provided to the individual 42 that travel to the destination is potentially unsafe. Different options may be presented to the individual 42 in such circumstances, such as choosing a different time to move through the building 22, a different beginning location or a different destination.

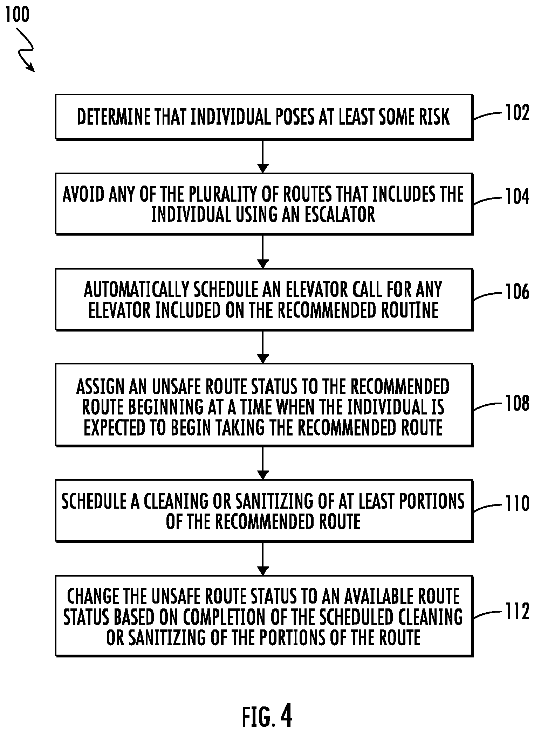

Figure 4 includes a flow chart diagram that summarizes an approach to determining a recommended route when the risk contribution of the individual 42 corresponds to that individual presenting additional or increased risk based on the characteristic(s) provided by the individual 42. For example, if the individual 42 indicates a known infection or contact tracing information indicates the individual was at high risk of recent exposure to the disease, the processor 34 determines at 102 that the individual 42 poses at least some risk. At 104, the processor 34 avoids any potential route that includes the individual 42 using an escalator. This feature addresses the challenges associated with cleaning or disinfecting the moving surfaces, such as the steps and handrail, of an escalator after an infected individual would ride the escalator. Cleaning a stairway or elevator tends to be simpler and requires less time during which the stairs or elevator car are out of service to be cleaned.

At 106, if there is an elevator 28 included on the recommended route determined at 60 in Figure 2, the processor 34 automatically initiates scheduling an elevator call for the individual 42. In some embodiments, such an elevator call includes isolating the individual 42 as the only person assigned to that elevator car.

Once the individual 42 presenting some risk has a recommend route, that route is assigned an unsafe status during a time the individual is expected to be on that route at 108. This avoids the processor 34 from considering that route for others. The illustrated example process includes scheduling cleaning or sanitizing at least portions of the recommended route at 110. This may be done by the processor 34 sending a communication to building cleaning personnel or otherwise generating a command for notice to be provided to an appropriate person or service.

Once the individual 42 arrives at the destination and any selected or necessary portions of the route have been cleaned, at 112, the processor 34 changes the status of the route from unsafe to available so it can be considered as a possible route for others.

The illustrated example embodiments facilitate minimizing risk to individuals who have to move between different levels in a building. As mentioned above, the risk of infection with a communicable disease is considered as an example scenario or type of risk. Other risks may be addressed using features of the disclosed embodiments by using appropriate criteria.

The features of the disclosed embodiments are not necessarily limited to the respective embodiments. Various combinations of the features of the example embodiments may be combined to realize additional embodiments.

The preceding description is exemplary rather than limiting in nature. Variations and modifications to the disclosed examples may become apparent to those skilled in the art that do not necessarily depart from the essence of this invention. The scope of legal protection given to this invention can only be determined by studying the following claims.

## Claims

1. A method of managing risk associated with at least one individual moving within a building, the method comprising:
determining a beginning location and a destination of an individual, wherein the beginning location and the destination are on different levels of the building;
determining a risk contribution of the individual based on an indication of at least one characteristic of the individual;
determining a plurality of routes between the beginning location and the destination;
determining a risk factor of each of the plurality of routes based on the risk contribution of the individual and other risk information regarding the respective routes; and
determining a recommended route from among the plurality of routes for the individual to arrive at the destination, wherein the risk factor of the recommended route is lower than the risk factor of at least one other one of the plurality of routes.

2. The method of claim 1, wherein
at least a first one of the plurality of routes includes an elevator and at least a second one of the plurality of routes includes at least one stairway or escalator, and
determining the recommended route includes prioritizing the first one of the routes based on the risk contribution of the individual satisfying at least one criterion.

3. The method of claim 2, comprising:
prioritizing the first one of the routes over the second one of the routes when the at least one criterion corresponds to the individual contributing to an increased risk of spreading infection within the building; and / or
when the first one of the routes is the recommended route, automatically scheduling a call for the elevator to take the individual along a corresponding portion of the recommended route to the destination.

4. The method of claim 1, 2 or 3, wherein
determining the risk contribution of the individual comprises receiving information regarding the at least one characteristic of the individual in association with receiving information regarding the beginning location and the destination, and
the at least one characteristic of the individual corresponds to a likelihood that the individual is infected with a contagious disease, optionally wherein the information regarding the at least one characteristic of the individual is based on an output of a contract tracing application.

5. The method of any preceding claim, wherein
the determined risk contribution of the individual indicates that it is unlikely that the individual has contracted a contagious disease,
the risk factor corresponds to a risk that the individual will be exposed to the contagious disease or will encounter at least one other individual that is likely infected with the contagious disease, and
the recommended route reduces or minimizes the risk that the individual will be exposed to the contagious disease while moving toward the destination,
optionally wherein the method comprises determining a relationship between the risk factor of the recommended route and at least one threshold;
when the risk factor of the recommended route is below a first threshold, notify the individual that travel to the destination is approved with low risk and automatically schedule an elevator call if an elevator is included on the recommended route;
when the risk factor of the recommended route is above the first threshold and below a second threshold,
notify the individual that travel to the destination at a current time has an associated risk of infection,
determine at least one alternative route at a different time,
notify the individual of the at least one alternative route, and
receive a selection from the individual of the recommended route or the alternative route; and
when the risk factor of the recommended route is above the second threshold, notify the individual that it is potentially unsafe for the individual to travel to the destination at the current time.

6. The method of any of claims 1 to 4, wherein
the determined risk contribution of the individual indicates that it is likely or certain that the individual has contracted a contagious disease,
the risk factor corresponds to a risk that the individual will expose others along the respective route to the contagious disease, and
the recommended route reduces or minimizes the risk that the individual will expose others to the contagious disease while moving toward the destination,
optionally wherein determining the recommended route comprises avoiding any of the plurality of routes that includes the individual using an escalator and the method comprises
automatically scheduling an elevator call for any elevator included on the recommended route;
assigning an unsafe route status to the recommended route beginning at a time when the individual is expected to begin taking the recommended route;
scheduling a cleaning or sanitizing of at least portions of the recommended route; and
changing the unsafe route status to an available route status based on completion of the scheduled cleaning or sanitizing of at least the portions of the recommended route.

7. A system for managing risk associated with at least one individual moving within a building, the system comprising:
a receiver configured to receive an indication of at least one characteristic of an individual and an indication of a beginning location and a destination of the individual, wherein the beginning location and the destination are on different levels of the building; and
a processor configured to
determine a risk contribution of the individual based on the indication of the at least one characteristic of the individual;
determine a plurality of routes between the beginning location and the destination;
determine a risk factor of each of the plurality of routes based on the risk contribution of the individual and other risk information regarding the respective routes; and
determine a recommended route from among the plurality of routes for the individual to arrive at the destination, wherein the risk factor of the recommended route is lower than the risk factor of at least one other one of the plurality of routes.

8. The system of claim 7, wherein
at least a first one of the plurality of routes includes an elevator and at least a second one of the plurality of routes includes at least one stairway or escalator, and
the processor is configured to determine the recommended route by prioritizing the first one of the routes based on the risk contribution of the individual satisfying at least one criterion.

9. The system of claim 8, wherein the at least one criterion corresponds to the individual contributing to an increased risk of spreading infection within the building, and /or
wherein, when the first one of the routes is the recommended route, the processor automatically schedules a call for the elevator to take the individual along a corresponding portion of the recommended route to the destination.

10. The system of any of claims 7 to 9, wherein the at least one characteristic of the individual corresponds to a likelihood that the individual is infected with a contagious disease.

11. The system of claim 10, wherein the information regarding the at least one characteristic of the individual is based on an output of a contract tracing application.

12. The system of any of claims 7 to 11, wherein
the determined risk contribution of the individual indicates that it is unlikely that the individual has contracted a contagious disease,
the risk factor corresponds to a risk that the individual will be exposed to the contagious disease or will encounter at least one other individual that is likely infected with the contagious disease, and
the recommended route reduces or minimizes the risk that the individual will be exposed to the contagious disease while moving toward the destination.

13. The system of claim 12, wherein the processor is configured to
determine a relationship between the risk factor of the recommended route and at least one threshold;
when the risk factor of the recommended route is below a first threshold, notify the individual that travel to the destination is approved with low risk and automatically schedule an elevator call if an elevator is included on the recommended route;
when the risk factor of the recommended route is above the first threshold and below a second threshold,
notify the individual that travel to the destination at a current time has an associated risk of infection,
determine at least one alternative route at a different time,
notify the individual of the at least one alternative route, and
receive a selection from the individual of the recommended route or the alternative route; and
when the risk factor of the recommended route is above the second threshold, notify the individual that it is potentially unsafe for the individual to travel to the destination at the current time.

14. The system of any of claims 7 to 11, wherein
the determined risk contribution of the individual indicates that it is likely or certain that the individual has contracted a contagious disease,
the risk factor corresponds to a risk that the individual will expose others along the respective route to the contagious disease, and
the recommended route reduces or minimizes the risk that the individual will expose others to the contagious disease while moving toward the destination.

15. The system of claim 14, wherein determining the recommended route comprises avoiding any of the plurality of routes that includes the individual using an escalator and the processor is configured to
automatically schedule an elevator call for any elevator included on the recommended route;
assign an unsafe route status to the recommended route beginning at a time when the individual is expected to begin taking the recommended route;
schedule a cleaning or sanitizing of at least portions of the recommended route; and
change the unsafe route status to an available route status based on completion of the scheduled cleaning or sanitizing of at least the portions of the recommended route.
